# EUROPEAN PATENT SPECIFICATION

(11) **EP 3 167 391 B1**
(45) Date of publication and mention of the grant of the patent: **26.02.2020**
(21) Application number: 15745610.4
(22) Date of filing: 01.07.2015
(51) Int. Cl.: A61J 1/03, A61J 7/00, A61J 7/04

(54) **METHODS AND SYSTEMS FOR MONITORING A PLURALITY OF MEDICATION-CONTAINING CELLS OF A MEDICATION CONTAINER**
VERFAHREN UND SYSTEME ZUR ÜBERWACHUNG EINER VIELZAHL VON MEDIKAMENTENHALTIGEN ZELLEN EINES MEDIKAMENTENBEHÄLTERS
PROCÉDÉS ET SYSTÈMES POUR SURVEILLER UNE PLURALITÉ DE CELLULES CONTENANT UN MÉDICAMENT D'UN RÉCIPIENT DE MÉDICAMENTS

(30) Priority: 08.07.2014 US 201462021772 P
(43) Date of publication of application: 17.05.2017
(73) Proprietor: Avery Dennison Retail Information Services, LLC, Westborough, MA 01581 (US)
(72) Inventor: FORSTER, Ian J., Chelmsford-Essex CM16LA (GB)
(74) Representative: Müller-Boré & Partner Patentanwälte PartG mbB
(86) International application number: PCT/US2015/038763
(87) International publication number: WO 2016/007344

(56) References cited:
- US-A- 4 526 474
- US-A- 5 412 372
- US-A1- 2014 039 445

## Description

### Cross-Reference to Related Application(s)

The present application claims priority from U.S. Provisional Application No. 61/923,947 filed July 8, 2014.

### Background

### Field of the Disclosure

The present subject matter relates to monitoring the medication intake of a subject. More particularly, the present subject matter relates to electronically monitoring a plurality of medication-containing cells of a medication container.

### Description of Related Art

Frequently, a doctor or medical care provider will issue instructions to a subject to periodically ingest one or more doses of medication in the form of a pill or tablet or capsule or the like as part of a treatment regimen. Unless the subject is within a facility under the control of the doctor or medical care provider (e.g., a hospital or nursing home), it can be difficult for the doctor or medical care provider to know whether the subject is ingesting the prescribed medication at the proper times. Accordingly, it would be advantageous to provide systems and methods that allow a doctor or medical care provider to monitor the medication compliance of a subject, particularly when the subject is instructed to ingest several doses of medication from a medication container of the type having a plurality of medication-containing cells (e.g., a blister pack). US 5 412 372 A discloses portable medication dispenser that aids and monitors regimen-conforming use of medications provided in standard as-marketed blister packages. A standard blister package and a separate, disposable sensor sheet are simply and easily loaded into a compact housing containing supervisory electronics, visual indicators, and an audible alarm. The sensor sheet consists of electrical or optical sensing regions corresponding to each individual compartment of the blister package. A compliant, electrically conductive or optical connector in the dispenser housing allows periodic scanning of the sensor sheet circuitry by the control circuitry in the dispenser housing without adding connector components to the low cost, disposable sensor sheet. When a medication is ejected through the backing layer of the standard blister package, an electrical or optical parameter associated with a corresponding region of the sensor sheet is altered, thereby permitting the control circuitry to monitor the dispensing event. Stored patient compliance data may then be reported remotely through an optical or electrical interface between the dispenser and an external system for review by the pharmacist/physician.

US 4 526 474 A discloses a device for storing, and periodically announcing the time for removal of, drug doses in pill, tablet or capsule form. A patient is warned at regular intervals to remove a drug dose from a blister pack serving as drug container. An electronic timer system associated with the blister pack activates periodically a signal emitter which emits a preferably acoustic signal. Optionally an optical signal emitter can also be installed in the warning system. The system comprises an electrically activatable input which receives a starting pulse at the time when a first drug dose is to be removed from the blister pack. The starting pulse is generated by rupturing, through removal of a drug dose from a blister pocket, an electric pulse lead which extends across the blister pocket in the rupturable closing foil of the latter and which is connected to the starting pulse input of the timer in the warning system.

US 2014/039445 A1 discloses a computer-based system for implementing and monitoring a medication regimen, including a medication package and a computer-based unit. The package includes a base including spaces for receiving a medication, a sheet of material including respective portions sealing the spaces with respect to the base, and a respective electrical circuit associated with each portion. The computer-based unit includes a memory element to store computer readable instructions and a medication regimen, formulated by a medical practitioner or pharmacist, for the medication, a processor, and a wireless transmitter. The computer-based unit is attachable to the medication package or can wirelessly communicate with the medication package. The processor executes the computer readable instructions to: monitor the electrical circuits to detect when portions are ruptured, and wirelessly transmit, using the wireless transmitter, a compliance message for receipt by at least one computer, the compliance message indicating whether a portion has been ruptured.

### Summary

There are several aspects of the present subject matter which may be embodied separately or together in the devices and systems described and claimed below. These aspects may be employed alone or in combination with other aspects of the subject matter described herein.

In one aspect, a system for monitoring usage of a medication container having a plurality of medication-containing cells includes an electrical circuit and a measurement device. The circuit includes a plurality of sequential value element electrically coupled in parallel between ground and a single port. Each sequential value element is configured for association with a different one of the cells and has a unique value. The circuit also includes a wireless supply, to provide power such that a separate power source is omitted. The circuit also includes a plurality of frangible sections, with each frangible section being configured for association with a different one of the cells and a different one of the sequential value elements. Each frangible section is configured to be broken to access medication within the associated cell and to electrically uncouple the associated sequential value element from the electrical circuit. The measurement device is associated with the port and programmed to calculate the combined value of the sequential value elements electrically coupled to the electrical circuit. The measurement device then determines, based on the combined value, which of the cells have been accessed via breakage of the associated frangible section.

In another aspect, a system for monitoring medication usage includes a medication container, an electrical circuit, and a measurement device. The medication container has a plurality of medication-containing cells. The circuit includes a plurality of sequential value element electrically coupled in parallel between ground and a single port. Each sequential value element is associated with a different one of the cells and has a unique value. The circuit also includes a plurality of frangible sections, with each frangible section being associated with a different one of the cells and a different one of the sequential value elements. Each frangible section is configured to be broken to access medication within the associated cell and to electrically uncouple the associated sequential value element from the electrical circuit. The measurement device is associated with the port and programmed to calculate the combined value of the sequential value elements electrically coupled to the electrical circuit. The measurement device then determines, based on the combined value, which of the cells have been accessed via breakage of the associated frangible section.

In yet another aspect, a method of monitoring medication usage includes associating an electrical circuit and a medication container having a plurality of medication-containing cells. The circuit and container are associated such that each cell has an associated sequential value element of the electrical circuit and an associated frangible section. Each sequential value element has a unique value and is electrically coupled to the electrical circuit by the associated frangible section which, when broken, uncouples the associated sequential value element from the electrical circuit and allows access to medication within the associated cell. The combined value of the sequential value elements electrically coupled to the electrical circuit is calculated and, based on the combined value, it is determined which of the cells have been accessed via breakage of the associated frangible section. The method of monitoring medication usage also has a wireless supply, to provide power such that a separate power source is omitted.

In yet another aspect, a method of monitoring medication usage includes associating an electrical circuit and a medication container which has a plurality of medication-containing cells. The circuit and container are associated such that each cell has a substantially equal value element of the electrical circuit and an associated frangible section. Each element has the same value and is electrically coupled to the electrical circuit by the associated frangible section which, when broken, uncouples the associated element from the electrical circuit and allows access to medication within the associated cell. The combined value of the elements electrically coupled to the electrical circuit is calculated and, based on the combined value, it is determined how many of the cells have been accessed via breakage of the associated frangible section, and, if monitored against time, when each cell was opened. If the medication in the cells are the same, one does not actually need to know which cell was opened at a given time, simply that a cell has been opened; this makes the circuitry potentially simpler, as all the elements associated with the cells can be the same, which are easier to fabricate.

### Brief Description of the Drawings

Fig. 1 is a diagrammatic view of a circuit for electrically coupling a medication container to an electronic device having a capacitive touchscreen according to an aspect of the present disclosure;
Fig. 2 is a side elevational view of a system in which a medication container and an electronic device having a capacitive touchscreen are electrically coupled to monitor medication compliance;
Fig. 3 is a detail view of a medication-containing cell of the medication container of Fig. 2;
Fig. 4 is a top plan view of the system of Fig. 2;
Fig. 5 is a top plan view of the medication container of Fig. 2;
Fig. 6 is a bottom plan view of the medication container of Fig. 2;
Fig. 7 is a side elevational view of another embodiment of a system in which a medication container and an electronic device having a capacitive touchscreen are electrically coupled to monitor medication compliance;
Fig. 8 is a perspective view of a system in which a medication container and an electronic device having a capacitive touchscreen are electrically coupled using an adapter to monitor medication compliance;
Fig. 9 is a diagrammatic view of another alternative embodiment of an electrical circuit for monitoring the cells of a medication container;
Figs. 10-14 are diagrammatic views of electrical components that may be incorporated into a circuit of the type shown in Fig. 9;
Fig. 15 is a diagrammatic view of a remote monitoring system incorporating a circuit of the type shown in Fig. 9; and
Fig. 16 is a diagrammatic view of a system in which several circuits of the type shown in Fig. 9 are multiplexed together.

### Description of the Illustrated Embodiments

As required, detailed embodiments of the present invention are disclosed herein; however, it is to be understood that the disclosed embodiments are merely exemplary of the invention, which may be embodied in various forms.

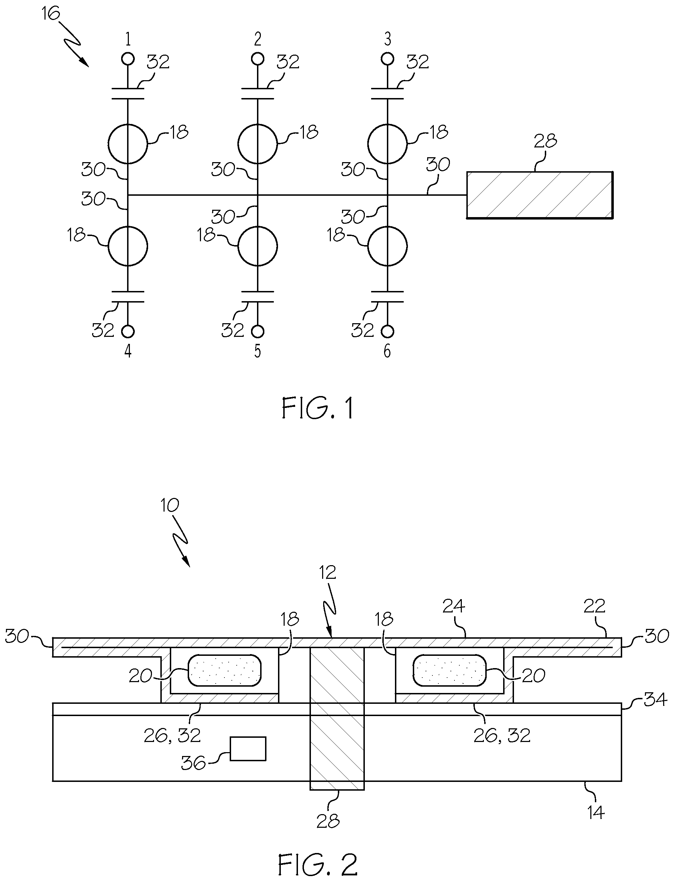

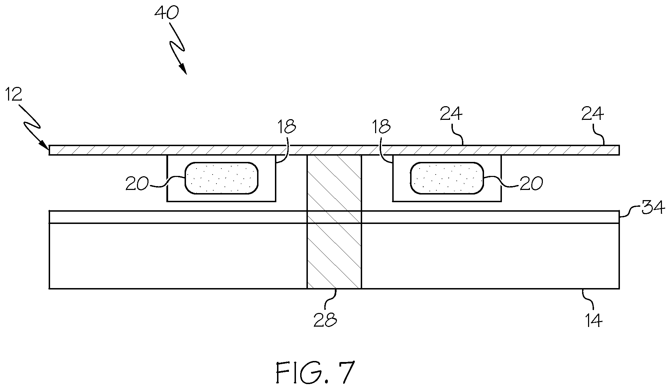

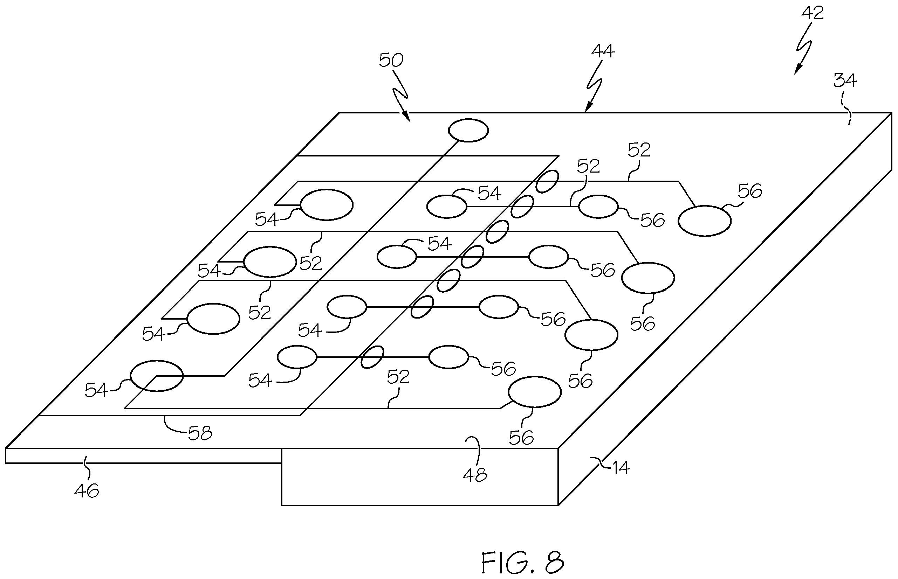

According to an aspect of the present disclosure, a doctor or medical care provider may remotely monitor medication compliance through the use of a system 10 which combines a medication container 12 and an electronic device 14. Fig. 1 illustrates an exemplary circuit 16 by which the status of a medication-containing cell 18 of a medication container 12 may be monitored using an electronic device 14 that is electronically coupled to the medication container. Figs. 2-6 illustrate one embodiment of such a system 10, while Figs. 7 and 8 illustrate two alternative embodiments of such a system.

In the embodiment of Figs. 1-6, a medication container 12 includes at least one cell 18 in which a medication 20 is contained. Preferably, the medication container 12 includes a plurality of identical cells 18, but it is also within the scope of the present disclosure for a medication container to include differently configured cells or only one cell.

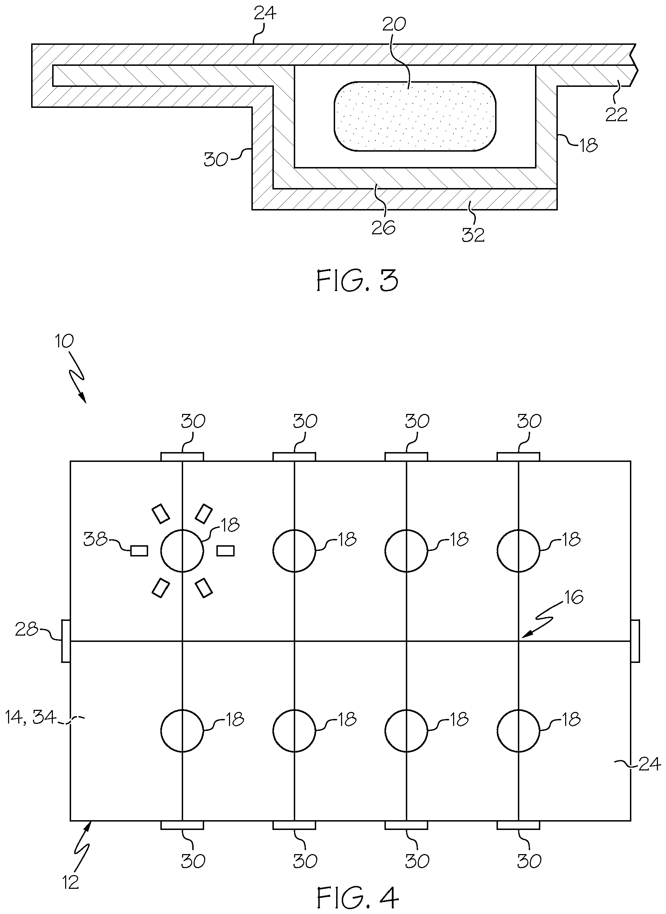

Each cell 18 may be formed of any suitable material but, in one embodiment, each cell is formed of a plastic material or another material that is substantially non-conductive. It may be advantageous for the cells 18 to be formed of a material that is generally rigid, but sufficiently deformable that a human may deform the individual cells using a finger or digital force and manipulation. In a preferred embodiment, the body of the medical container 12 takes the general form of a blister pack, with a thin plastic sheet 22 (Figs. 2 and 3) being provided with a plurality of vacuum-formed depressions or formations that each defines a cell 18 for receiving a dose of medication 20. While it may be preferred for a medication container having a plurality of cells to be provided with a single plastic sheet that is formed to define all of the cells, it is also within the scope of the present disclosure for the cells of a single medication container to be separately or non-integrally formed.

Each cell 18 is closed or overlaid by a cover 24 through which medication 20 within the cell 18 may be accessed. In one embodiment, the cover 24 is a thin sheet of material, such as a metallic foil or an at least partially light-transmissive (e.g., transparent or translucent) sheet, which may be broken to allow a medication 20 to pass out of the cell 18. In such an embodiment, a base 26 of the cell 18 may be pressed toward the frangible cover 24 by a user until the force on the cover 24 exceeds the strength of the cover 24, at which point the cover 24 breaks and the medication 20 may be removed from the cell 18. Alternatively, the cell 18 may remain untouched, while the cover 24 is directly engaged and broken to remove medication 20 from the cell 18. If the medication container 12 is provided with a plurality of cells 18, it may be preferred for a single cover 24 to overlay all of the cells 18, but it is also within the scope of the present disclosure for two or more cells of the same medication container to be provided with separate covers. For example, in one embodiment, different cells are each overlaid by separate, non-frangible (e.g., hinged) covers.

In the embodiment of Figs. 1-6, a circuit 16 is incorporated into the medication container 12. The circuit 16 of Fig. 1 includes a tab or conductor 28 that provides a virtual ground, as will be described in greater detail herein. A conductor 30 associated with the cover 24 extends from the tab 28 to cross over the cell 18. If the medication container 12 is provided with a plurality of cells 18, as in the illustrated embodiment, a single conductor 30 may be configured to cross over each of the cells 18, but it is also within the scope of the present disclosure for separate cells to have separate conductors associated therewith. In a particular embodiment, the conductor 30 may be printed onto the cover 24 or otherwise integrated into the cover 24, but it also within the scope of the present disclosure for the conductor to be separately provided from the cover.

The illustrated circuit 16 further includes a capacitor 32 that is electrically coupled to the tab 28 by the conductor 30, with the portion of the conductor 30 passing over the cell 18 positioned between the tab 28 and the capacitor 32. In the illustrated embodiment, the tab 28 is associated with the same surface of the medication container 12 as the cover 24, which is opposite the surface of the medication container 12 where the bases 26 of the cells 18 are located. In the orientation of Fig. 2, the upper surface of the medication container 12 is shown as being associated with the cover 24, while the cells 18 extend downwardly from the lower surface of the medication container 12. In the illustrated embodiment, the conductor 30 is wrapped around the edge of the medication container 12 to place the capacitor 32 on the lower surface, preferably directly below the cell 18. As best shown in Fig. 2, so positioning the capacitor 32 at the base 26 of the cell 18 places it into contact with the capacitive touchscreen 34 of an associated electronic device 14, thereby creating a virtual touch point, when the medication container 12 is mounted onto the electronic device 14, as will be described in greater detail herein.

The electronic device 14 of the system 10 may be variously configured, provided that it includes a capacitive touchscreen 34. For example, the electronic device 14 may be a mobile phone or a tablet computer or a controller of an electronic gaming device or the like. Preferably, the capacitive touchscreen 34 is of the type that may detect multiple touch points simultaneously in order to accommodate a medication container 12 with a plurality of cells 18, as in the illustrated embodiment.

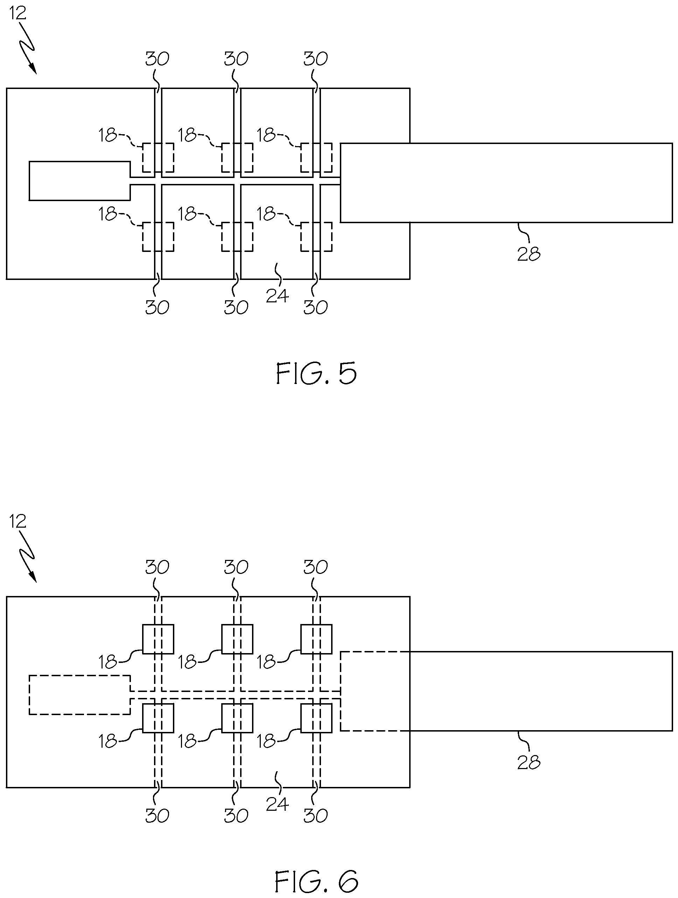

In use, a medication container 12 having a circuit 16 of the type shown in Fig. 1 is provided, as in Figs. 5 and 6. The medication container 12 is connected to the electronic device 14 so as to place the base 26 of the cell or cells 18 against the capacitive touchscreen 34 (Fig. 2) of the electronic device 14, thereby bringing the capacitor or capacitors 32 of the circuit 16 into contact with the capacitive touchscreen 34, creating one or more virtual touch points. The tab 28 of the circuit 16 may be at least partially wrapped around the edge of the electronic device 14 to contact an insulated portion thereof (Fig. 2), creating a virtual ground for the circuit 16. Additional means may also be provided for securing the medication container 12 to the electronic device 14, as need be. Alternatively, in one embodiment, the tab 28 may be the sole means by which the medication container 12 is connected to the electronic device 14, with the tab 28 being sufficiently flexible to define a hinge that allows the medication container 12 to be temporarily swung or pivoted away from the capacitive touchscreen 34 for accessing the medication 20, before swinging or pivoting the medication container 12 back into position against the capacitive touchscreen 34.

With the circuit 16 of the medication container 12 creating at least one touch point on the capacitive touchscreen 34, the electronic device 14 is effectively electrically coupled to the medication container 12. As such, the electronic device 14 is able to detect the presence of the cell 18 and register it as containing a medication 20, with the cover 24 intact over the cell 18. In one embodiment, the electronic device 14 may be programmed to continuously monitor the status of the cell or cells 18. In another embodiment, the electronic device 14 may include an accelerometer 36 and may be programmed to monitor the status of the cell or cells 18 only when the accelerometer 36 detects movement of the electronic device 14 (e.g., when a subject picks up the system 10 to remove medication 20 from a cell 18).

Accessing a cell 18 through the cover 24 to remove the medication 20 disrupts the circuit 16 at the location of the cell 18, for example by severing or breaking the conductor 30 at the cell 18. By so changing the structure of the circuit 16, the signal being received by the electronic device 14 from the cell 18 via the capacitive touchscreen 34 will change. The electronic device 14 may be programmed to register such a change in (or absence of) the signal being received by the capacitive screen 34 at the touch point corresponding to the location of the cell 18 as an indication that the medication 20 has been removed from the cell 18. If the medication container 12 includes a plurality of cells 18, the electronic device 14 may separately monitor the status of each cell 18 and distinguish them based on their different locations with respect to the capacitive touchscreen 34. This may be especially advantageous if the various cells contain different medications that are to be ingested by the subject at particular times.

The electronic device 14 may be programmed to respond to the change at the touch point in any of a number of ways. For example, it may send a signal to the doctor or medical care provider to indicate that a particular medication was taken by the subject at a particular date and time. In another embodiment, the electronic device 14 may store information about the event in an internal database or in an external storage location, which may be later accessed by the doctor or medical care provider.

In addition to responding to a change at a virtual touch point, the electronic device 14 may be programmed to execute any of a number of other operations in connection with medication monitoring. For example, if the medication container 12 is at least partially light-transmissive, then the electronic device 14 may be programmed to display one or more visual indications 38 (Fig. 4) adjacent to a cell 18 to alert the subject that it is time to access the cell 18 and ingest the medication 20 contained therein or to perform some other action. Alternatively, if the medication container 12 and/or cover 24 are formed of a substantially opaque material, the medication container 12 and/or cover 24 may include voids or apertures through which the underlying capacitive touchscreen 34 may be seen in order to allow the electronic device 14 to visually alert the subject. Additionally, or alternatively, the electronic device 14 may be programmed to emit an alarm or other audible signal (such as recorded speech) and/or vibrate to alert the subject that it is time to take a dose of medication. The electronic device 14 may also be programmed to provide the subject with a direct voice link to the doctor or medical care provider (either initiated by the doctor/medical care provider to offer guidance or by the subject to request assistance) when accessing the medication 20.

In an alternative embodiment not covered by the claims, which is shown in Fig. 7, a different mechanism is used to ascertain whether a cell 18 has been accessed through the cover 24 of the medication container 12 to remove the medication 20. In particular, the dielectric constant "k" of the medication 20 differs from that of the cover 24 (particularly, the circuit incorporated into the cover 24), such that there is a localized difference in capacitance at the location of a cell 18 containing a dose of medication 20. For example, in one embodiment, calcium carbonate has a dielectric constant of 9.1, which causes a localized increase in the capacitance at the location of the associated cell 18. When the cell 18 is accessed through the cover 24 and the medication 20 is removed, the capacitance at the cell 18 will change (decrease in the case of calcium carbonate), which changes the signal received by the electronic device 14. The electronic device 14 may be programmed to recognize a change in the localized capacitance as an indication that the medication 20 has been removed from the cell 18 positioned at the location of the changed capacitance. As in the embodiment, of Figs. 2-6, the system 40 of Fig. 7 may be configured to accommodate several cells 18, with the electronic device 14 being programmed to monitor the status of all of the cells 18 and pinpoint which particular cell has been accessed through the cover 24 by a subject.

For some subjects, a system in which the cells of the medication container are positioned at a location that is offset from the capacitive touchscreen may be preferred to a system in which the cells are positioned in contact with the capacitive touchscreen and accessed with the cells in that position or after temporarily moving the medication container out of contact with the capacitive touchscreen. Subjects may also prefer a system not covered by the claims in which a standard medication container, rather than a medication container of the type described herein with an incorporated circuit, is used in combination with an electronic device. To that end, another embodiment of a system 42 according to the present disclosure may include an adaptor or case 44 that electrically couples a standard medication container to an electronic device 14 having a capacitive touchscreen 14 (Fig. 8).

In the illustrated embodiment, the adaptor 44 has a first portion 46 that is positioned laterally or otherwise offset from a second portion 48 of the adaptor 44. The first portion 46 is configured to receive at least a portion of a medication container having one or more medication-containing cells, while the second portion 48 is configured to be placed adjacent to the capacitive touchscreen 34 of an electronic device 14. The adaptor 44 includes a circuit 50 with one or more conductors 52 extending between the first portion 46 and the second portion 48. For each cell of the medication container, the first portion 46 of the adaptor 44 may include a corresponding cell connection point 54, with a conductor 50 extending between the cell connection point 54 and a corresponding touchscreen connection point 56 at the second portion 48 of the adaptor 44.

When the second portion 48 of the adaptor 44 is secured or connected to the electronic device 14, each touchscreen connection point 56 engages the capacitive touchscreen 34 of the electronic device. When a medication container is associated with the first portion 46 of the adaptor 44, such as by sliding the medication container into a slot 58 defined in the first portion 46, each cell will be positioned at a corresponding cell connection point 54. On account of the conductor 52 extending between each cell connection point 54 and the corresponding touchscreen connection point 56, connecting both the medication container and the electronic device 14 to the adaptor 44 electrically couples each cell of the medication container to a corresponding location of capacitive touchscreen 34, creating one or more virtual touch points on the capacitive touchscreen 34. With both the medication container and the electronic device 14 connected to the adaptor 44, the system 42 may be used to detect whether a cell of the medication container has been accessed through the cover of the medication container according to the principles described above with respect to the embodiments of Figs. 1-6 and 7.

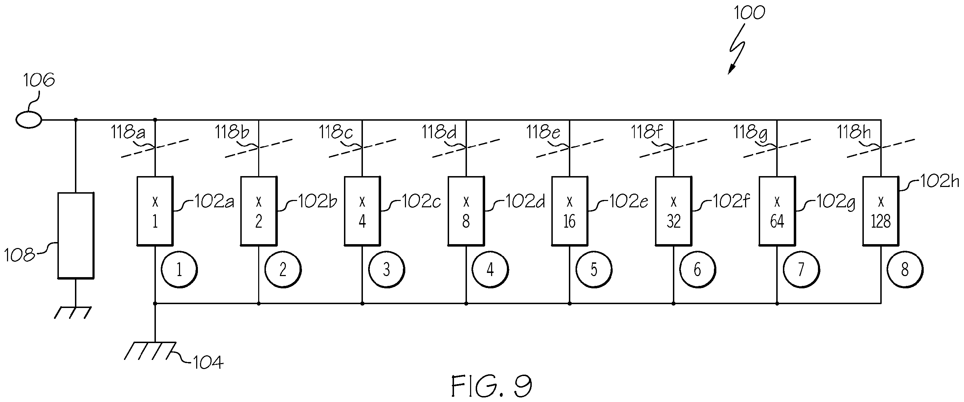

Fig. 9 illustrates an alternative circuit 100 that may be associated with a medication container having a plurality of medication-containing cells. The electrical circuit 100 includes a plurality of sequential value elements 102a, 102b, 102c, 102d, 102e, 102f, 102g and 102h that are electrically coupled in parallel between ground 104 and a single communication port 106. The electrical circuit 100 may include additional components (e.g., a parasitic constant element 108) without departing from the scope of the present disclosure.

Preferably, the number of sequential value elements is equal to the number of cells of the medication container, with each sequential value element being associated with or assigned to a different one of the cells. For example, in the illustrated embodiment, the circuit 100 includes eight sequential value elements 102a-102h, which would preferably be used in combination with a medication container having eight medication-containing cells. While it is preferred for the number of sequential value elements to match the number of cells, it is within the scope of the present disclosure for there to be more sequential value elements than cells or for there to be more cells than sequential value elements.

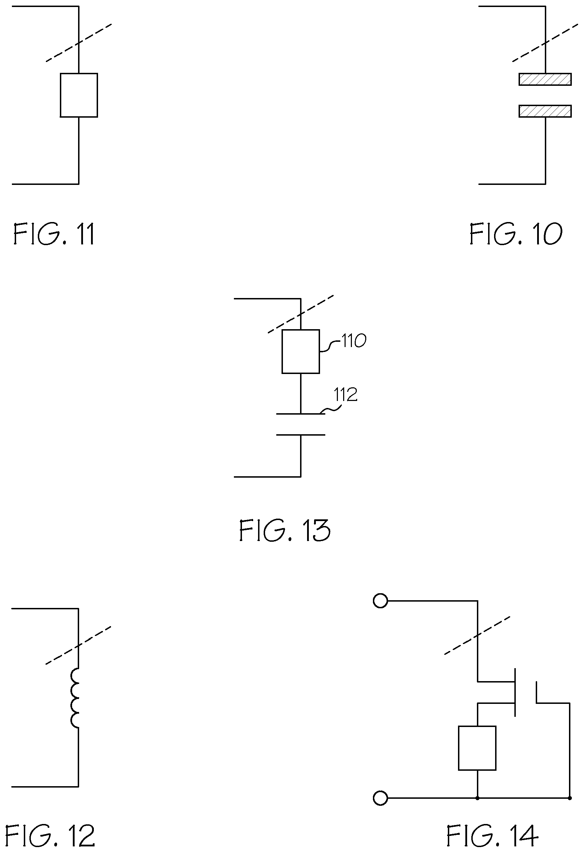

The sequential value elements 102a-102h may be variously configured without departing from the scope of the present disclosure. For example, in one embodiment, the sequential value elements 102a-102h may each be provided as a capacitor (Fig. 10). In other embodiments, the sequential value elements 102a-102h may each be provided as resistors (Fig. 11) or inductors (Fig. 12). In another embodiment, the sequential value elements 102a-102h may each be provided as a combination of electrical components, as in Fig. 13, where a sequential value element is provided as a resistor 110 in series with a capacitor 112. In yet another embodiment, the sequential value elements may each be provided as an electrical component having semi-conductive properties, such as a printed transistor (Fig. 14), which may act as a current sink. Other configurations of the sequential value elements 102a-102h are possible without departing from the scope of the present disclosure.

Each sequential value element may have a unique value, allowing the sequential value elements to be distinguished from each other. For example, Fig. 9 illustrates an embodiment in which a set of sequential value elements have values that increase in a binary sequence, from 1 (sequential value element 102a) to 2 (sequential value element 102b) to 4 (sequential value element 102c) to 8 (sequential value element 102d) to 16 (sequential value element 102e) to 32 (sequential value element 102f) to 64 (sequential value element 102g) to 128 (sequential value element 102h). While a set of sequential value elements 102a-102h having values that increase in a binary sequence is illustrated, other unique values may be assigned to the different sequential value elements of a circuit and/or different sequential patterns may be employed to select the unique values of the different sequential value elements. The binary sequence can also be achieved by using a series of shunt and series elements, a structure commonly described as an "R2R" (resistor) ladder, where only two values of resistor are required, "R" and twice that value. This is an advantage were a comparatively large number of cells are required to be monitored, for example 16, where in the simple binary case cell 16 will have an associated resistor or capacitor 32768 times the element associated with cell 1.

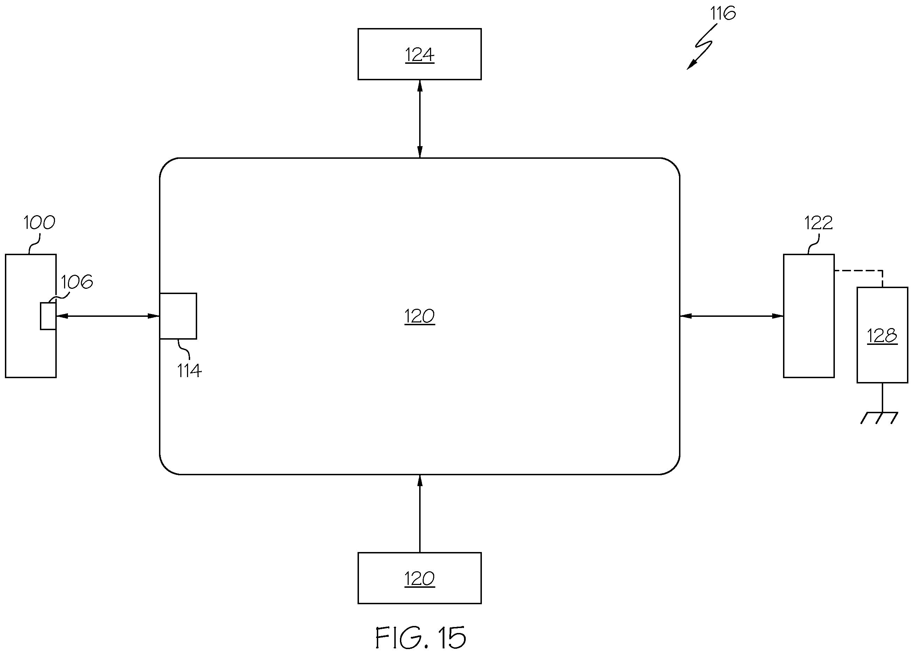

A measurement device 114 (such as a microcontroller) of a system 116 incorporating a circuit 100 of the type shown in Fig. 9 (Fig. 15) may be associated with the circuit 100 via the port 106 and programmed to calculate the combined value (i.e., the sum) of the unique values of the sequential value elements electrically coupled to the circuit 100. In the illustrated embodiment, the combined value of the sequential value elements is 255 when all of the sequential value elements are electrically coupled to the circuit 100 (i.e., 1+2+4+8+16+32+64+128). In contrast, when one or more of the sequential value elements is uncoupled from the circuit 100, the combined value will be less than the maximum possible value. For example, if the first and eighth sequential value elements 102a and 102h are electrically uncoupled from the circuit 100, then the combined value of the sequential value elements 102b-102g still coupled to the circuit 100 will be 126 (i.e., 2+4+8+16+32+64 or 126). Based on the calculated combined value, the measurement device 106 may determine which of the sequential value elements 102a-102h are coupled to the circuit 100 and which are uncoupled from the circuit 100.

Depending on the nature of the sequential value elements 102a-102h, the significance of the calculated combined value and the way in which it is calculated may vary. For example, in an exemplary embodiment in which all of the sequential value elements 102a-102h are capacitors, the measurement device 114 may calculate the capacitance of the circuit 100, including the sequential value elements coupled to the circuit 100. In particular, the total capacitance will be equal to a zero value (which is the capacitance of the circuit 100 itself when there are no sequential value elements coupled to the circuit 100) plus the capacitance of the sequential value elements coupled to the circuit 100. In the illustrated embodiment, when all of the sequential value elements 102a-102h are coupled to the circuit 100, the capacitance of the circuit 100 will be equal to the zero value plus the sum of the individual values of the sequential value elements 102a-102h (255 or 1+2+4+8+16+32+64+128) multiplied by the value of the first or base sequential value element 102a (which has an individual value of 1 in the illustrated embodiment). If one or more of the sequential value elements 102a-102h is uncoupled from the circuit 100, then the capacitance of the circuit 100 will be somewhere between the zero value and the maximum value. For example, if the measurement device 114 measures a capacitance that is equal to the zero value plus 24 times the value of the first sequential value element 102a, then it can determine that only the fourth and fifth sequential value elements 102d and 102e are electrically coupled to the circuit 100, because their individual values are 8 and 16, respectively, resulting in a calculated combined value of 24.

If the sequential value elements 102a-102h are differently configured, then different steps may be taken by the measurement device 106 to determine which of the sequential value elements are coupled to the circuit 100. For example, if the sequential value elements 102a-102h are provided as resistors, then the measurement device 114 may be used to measure the resistance of the circuit 100, including the sequential value elements, and then determine which of the sequential value elements are coupled to the circuit 100 based on that measured resistance. If the sequential value elements 102a-102h are provided as a combination of multiple electrical components, then the measurement device 114 may be programmed to calculate one or more values, such as the resistance and/or the capacitance of the circuit 100 (including the sequential value elements coupled thereto), and use such information to determine which of the cells are coupled to and uncoupled from the circuit 100.

In the illustrated embodiment, each of the conductors coupling the individual sequential value elements 102a-102h to the circuit 100 is provided with a frangible section 118a, 118b, 118c, 118d, 118e, 118f, 118g and 118h. Each frangible section is configured to be broken to electrically uncouple the associated sequential value element from the circuit 100. Preferably, the location of each frangible section substantially coincides or is aligned with one of the cells of the medication container, such that accessing the cell to remove a dose of medication from the cell (e.g., by breaking the cover overlaying the cell) necessarily entails breaking the associated frangible section of the circuit 100. By such a configuration, it is possible to determine (based on measurements taken by the measurement device 114) how many cells of a medication container the patient has accessed, as well as the exact identity of the cell or cells that have been accessed. Such a configuration may be particularly advantageous because multiple cells may be monitored and individually identified without requiring more than one port or connection 106 to the measurement device 114, thereby decreasing the cost, size, and energy requirements of the system and making it more suitable for low-cost attachment techniques, such as the use of flip chips.

Fig. 15 illustrates a system 116 incorporating a circuit 100 of the type shown in Fig. 9, a measurement device 114, and a number of other optional components. In the embodiment of Fig. 15, the system 116 includes a controller 120 that coordinates the operation of the various other components of the system 116. The measurement device 114 may be incorporated into the controller 120 (as shown in Fig. 15) or it may be provided as a separate device.

The controller 120 may interact with a variety of devices, such as a power source 122, a communication device 124, and a timing device 126. The power source 122 may be variously provided, such as a battery or the like, provided with or without an associated storage device 128, such as a capacitor.

The communication device 124, if provided, may allow for the information measured and/or calculated by the measuring device 114 and/or controller 120 to be relayed to a doctor or medical care provider, thereby allowing remote monitoring of the medication usage of the patient. The communication device 124 may allow for communication by any of a number of ways, such as near-field communication ("NFC") wireless, Bluetooth, WiFi, contact or UHF data link or the like. In one embodiment, power may be drawn from a wireless supply, such as a read field for an NFC interface, in which case a separate power source 122 may be omitted.

If provided, the timing device 126 (such as one of the type described above in greater detail) may be used to determine when a particular cell was accessed, which information may be stored and/or transmitted to a doctor or medical care provider by the communication device 124. In another embodiment, the measurement device 114 may periodically monitor the status of the circuit 100, with the status of the circuit 100 being periodically transmitted by the communication device 124 or only upon recognition of a change in the status of the circuit 100 (e.g., when a cell has been accessed). The time at which the information is transmitted by the communication device 124 may be used to determine when a particular cell was accessed by the patient.

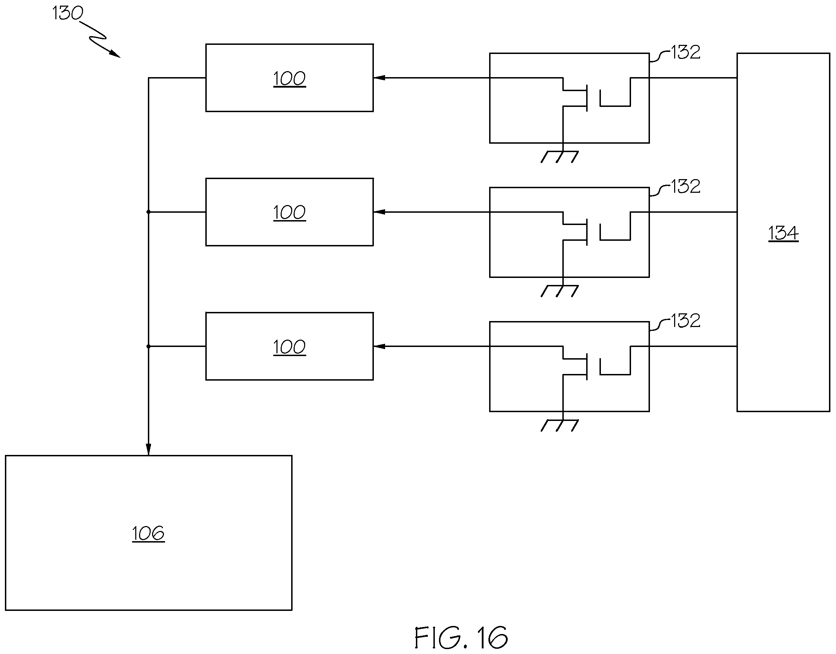

If the medication container has a relatively large number of cells, the unique values of the sequential value elements may become sufficiently large that it may become more difficult for a measurement device to make precise measurements. For example, if a medication container has ten cells, with an associated electrical circuit having ten corresponding sequential value elements with unique values that increase in a binary sequence, then the largest value will be 512 times the smallest value. For a medication container having more cells and an associated electrical circuit having more sequential value elements, the range in unique values will be even greater. Accordingly, to promote measurement accuracy, two or more electrical circuits 100 of the type shown in Fig. 9 may be employed in combination with a single medication container having a great number of cells. For example, Fig. 16 illustrates a system 130 in which three electrical circuits 100 are multiplexed together in parallel for use with a single medication container, with the circuits 100 sharing a single common port 106 that may communicate with a measurement device, as described above.

In the system 130 of Fig. 16, each electrical circuit 100 is provided as in Fig. 9, except with a transistor 132 positioned between the circuit 100 and ground. Each transistor 132 is configured to be switched between two states having different resistance, with one state having a low resistance and the other having a very high resistance. When a transistor 132 has been switched to the high resistance state, it may be considered to be an open circuit, such that the associated electrical circuit 100 will be treated as being uncoupled from the port 106. Accordingly, the transistors 132 may be selectively switched between their two states to place only one of them in the low resistance state at a time. The one electrical circuit 100 having the associated transistor 132 in its low resistance state will be treated as the only circuit coupled to the port 106, and a measurement device associated with the port 106 may observe and measure that circuit 100 to determine which of the cells associated with the sequential value elements of the circuit 100 have been accessed, as described above in greater detail.

The states of the transistors 132 may be switched by any suitable means or control device or switch 134 to periodically place each of the circuits 100 into sole communication with the port 106, thereby allowing a measurement device associated with the port 106 to monitor each circuit 100 in turn and individually. The measurement device and/or a controller associated with the measurement device may monitor the status of the transistors 132 to determine which of the circuits 100 is "active" (i.e., which circuit 100 has its associated transistor 132 in a low resistance state), which must be known to ascertain the identity of the circuit 100 and, hence, the cells being monitored. By such a system, the status of a great number of medication-containing cells of a medication container may be monitored without unduly increasing the range or magnitude of values of the sequential value elements. For example, in the illustrated embodiment, the individual cells of a medication container having twenty-four cells may be monitored using a maximum unique value of 128 (i.e., the maximum value of a sequential value element in an eight-element circuit in which the values of the elements increase in a binary sequence, as in Fig. 9), whereas a single circuit having twenty-four sequential value elements whose values increase in a binary sequence would have a maximum unique value of 8,388,608.

It will be understood that the embodiments described above are illustrative of some of the applications of the principles of the present subject matter. For these reasons, the scope hereof is not limited to the above description but is as set forth in the following claims.

## Claims

1. A system for monitoring usage of a medication container having a plurality of medication-containing cells, comprising:
an electrical circuit including
a plurality of sequential value elements electrically coupled in parallel between ground and a single port, with each sequential value element being configured for association with a different one of said plurality of medication-containing cells and having a unique value; and
a wireless supply, to provide power such that a separate power source is omitted; and
a plurality of frangible sections, with each frangible section being configured for association with a different one of said plurality of medication-containing cells and a different one of said plurality of sequential value elements, wherein each frangible section is configured to be broken to access medication within the associated cell and to electrically uncouple the associated sequential value element from the electrical circuit; and
a measurement device associated with the port and programmed to calculate the combined value of the sequential value elements electrically coupled to the electrical circuit and, based on the combined value, determine which of the cells have been accessed via breakage of the associated frangible section.

2. The system of claim 1, wherein at least one of said sequential value elements comprises a capacitor.

3. The system of claim 1, wherein at least one of said sequential value elements comprises a resistor.

4. The system of claim 1, wherein at least one of said sequential value elements comprises an inductor.

5. The system of claim 1, wherein at least one of said sequential value elements comprises a combination of electrical components.

6. The system of claim 1, wherein at least one of said sequential value elements comprises a semiconductor.

7. The system of claim 1, further comprising a second electrical circuit including a plurality of sequential value elements electrically coupled in parallel between ground and said single port, with each sequential value element of the second electrical circuit being configured for association with a different one of said plurality of medication-containing cells and having a unique value, and
a plurality of frangible sections, with each frangible section of the second electrical circuit being configured for association with a different one of said plurality of medication-containing cells and a different one of said plurality of sequential value elements of the second electrical circuit, wherein
each frangible section of the second circuit is configured to be broken to access medication within the associated cell and to electrically uncouple the associated sequential value element from the second electrical circuit,
the electrical circuits are electrically coupled in parallel, and
at least one of the electrical circuits includes a transistor positioned intermediate the sequential value elements of said at least one of the electrical circuits and ground.

8. A method of monitoring medication usage, comprising:
associating an electrical circuit and a medication container having a plurality of medication-containing cells such that each medication-containing cell includes an associated sequential value element of the electrical circuit and an associated frangible section, with each sequential value element having a unique value and being electrically coupled to the electrical circuit by the associated frangible section which, when broken, uncouples the associated sequential value element from the electrical circuit and allows access to medication within the associated cell;
calculating the combined value of the sequential value elements electrically coupled to the electrical circuit and, based on the combined value, determining which of the cells have been accessed via breakage of the associated frangible section; and
having a wireless supply, to provide power such that a separate power source is omitted.

9. The method of claim 8, wherein said calculating the combined value of the sequential value elements includes calculating the combined capacitance of the sequential value elements electrically coupled to the electrical circuit.

10. The method of claim 8, wherein said calculating the combined value of the sequential value elements includes calculating the combined resistance of the sequential value elements electrically coupled to the electrical circuit.

11. The method of claim 8, wherein said calculating the combined value of the sequential value elements includes calculating the combined inductance of the sequential value elements electrically coupled to the electrical circuit.

12. The method of claim 8, wherein said calculating the combined value of the sequential value elements includes calculating the combined values of two or more electrical properties of the sequential value elements electrically coupled to the electrical circuit.

13. The method of claim 9, wherein said two or more electrical properties are resistance and capacitance.

## Patentansprüche

1. System zum Überwachen der Verwendung eines Medikamentenbehälters, der eine Vielzahl von medikamentenhaltigen Zellen aufweist, umfassend:
eine elektrische Schaltung, die umfasst:
eine Vielzahl von aufeinanderfolgenden Wertelementen, die zwischen Erde und einem einzelnen Anschluss elektrisch parallelgeschaltet sind, wobei jedes aufeinanderfolgende Wertelement für die Zuordnung zu einer anderen von der Vielzahl von medikamentenhaltigen Zellen ausgestaltet ist und einen einzigartigen Wert aufweist; und
eine drahtlose Versorgung, um Leistung derart bereitzustellen, dass eine separate Leistungsquelle entfällt; und
eine Vielzahl von zerbrechlichen Abschnitten, wobei jeder zerbrechliche Abschnitt für die Zuordnung zu einer anderen von der Vielzahl von medikamentenhaltigen Zellen und einem anderen von der Vielzahl von aufeinanderfolgenden Wertelementen ausgestaltet ist, wobei jeder zerbrechliche Abschnitt ausgestaltet ist, aufgebrochen zu werden, um auf das Medikament innerhalb der zugeordneten Zelle zuzugreifen und das zugeordnete aufeinanderfolgende Wertelement von der elektrischen Schaltung elektrisch zu entkoppeln; und
eine Messvorrichtung, die dem Anschluss zugeordnet und programmiert ist, den Gesamtwert der aufeinanderfolgenden Wertelemente, die elektrisch mit der elektrischen Schaltung gekoppelt sind, zu berechnen und basierend auf dem Gesamtwert zu bestimmen, auf welche der Zellen durch das Aufbrechen des zugeordneten zerbrechlichen Abschnitts zugegriffen wurde.

2. System nach Anspruch 1, wobei mindestens eines der aufeinanderfolgenden Wertelemente einen Kondensator umfasst.

3. System nach Anspruch 1, wobei mindestens eines der aufeinanderfolgenden Wertelemente einen Widerstand umfasst.

4. System nach Anspruch 1, wobei mindestens eines der aufeinanderfolgenden Wertelemente einen Induktor umfasst.

5. System nach Anspruch 1, wobei mindestens eines der aufeinanderfolgenden Wertelemente eine Kombination aus elektrischen Bauteilen umfasst.

6. System nach Anspruch 1, wobei mindestens eines der aufeinanderfolgenden Wertelemente einen Halbleiter umfasst.

7. System nach Anspruch 1, ferner eine zweite elektrische Schaltung umfassend, die umfasst:
eine Vielzahl von aufeinanderfolgenden Wertelementen, die zwischen Erde und dem einzelnen Anschluss elektrisch parallelgeschaltet sind, wobei jedes aufeinanderfolgende Wertelement der zweiten elektrischen Schaltung für die Zuordnung zu einer anderen von der Vielzahl von medikamentenhaltigen Zellen ausgestaltet ist und einen einzigartigen Wert aufweist, und
eine Vielzahl von zerbrechlichen Abschnitten, wobei jeder zerbrechliche Abschnitt der zweiten elektrischen Schaltung für die Zuordnung zu einer anderen von der Vielzahl von medikamentenhaltigen Zellen und einem anderen von der Vielzahl von aufeinanderfolgenden Wertelementen der zweiten elektrischen Schaltung ausgestaltet ist, wobei
jeder zerbrechliche Abschnitt der zweiten Schaltung ausgestaltet ist, aufgebrochen zu werden, um auf das Medikament innerhalb der zugeordneten Zelle zuzugreifen und das zugeordnete aufeinanderfolgende Wertelement von der zweiten elektrischen Schaltung elektrisch zu entkoppeln,
die elektrischen Schaltungen elektrisch parallelgeschaltet sind, und
mindestens eine der elektrischen Schaltungen einen Transistor umfasst, der zwischen den aufeinanderfolgenden Wertelementen der mindestens einen der elektrischen Schaltungen und Erde angeordnet ist.

8. Verfahren des Überwachens der Medikamentenverwendung umfassend:
Zuordnen einer elektrischen Schaltung und eines Medikamentenbehälters, der eine Vielzahl von medikamentenhaltigen Zellen aufweist, sodass jede medikamentenhaltige Zelle ein zugeordnetes aufeinanderfolgendes Wertelement der elektrischen Schaltung und einen zugeordneten zerbrechlichen Abschnitt umfasst, wobei jedes aufeinanderfolgende Wertelement einen einzigartigen Wert aufweist und durch den zugeordneten zerbrechlichen Abschnitt elektrisch mit der elektrischen Schaltung gekoppelt ist, der, wenn aufgebrochen, das zugeordnete aufeinanderfolgende Wertelement von der elektrischen Schaltung entkoppelt und den Zugang zum Medikament innerhalb der zugeordneten Zelle erlaubt;
Berechnen des Gesamtwerts der aufeinanderfolgenden Wertelemente, die elektrisch mit der elektrischen Schaltung gekoppelt sind, und, basierend auf dem Gesamtwert, Bestimmen, auf welche der Zellen durch das Aufbrechen des zugeordneten zerbrechlichen Abschnitts zugegriffen wurde; und
Aufweisen einer drahtlosen Versorgung, um Leistung derart bereitzustellen, dass eine separate Leistungsquelle entfällt.

9. Verfahren nach Anspruch 8, wobei das Berechnen des Gesamtwerts der aufeinanderfolgenden Wertelemente Berechnen der Gesamtkapazität der aufeinanderfolgenden Wertelemente umfasst, die elektrisch mit der elektrischen Schaltung gekoppelt sind.

10. Verfahren nach Anspruch 8, wobei das Berechnen des Gesamtwerts der aufeinanderfolgenden Wertelemente Berechnen des Gesamtwiderstands der aufeinanderfolgenden Wertelemente umfasst, die elektrisch mit der elektrischen Schaltung gekoppelt sind.

11. Verfahren nach Anspruch 8, wobei das Berechnen des Gesamtwerts der aufeinanderfolgenden Wertelemente Berechnen der Gesamtinduktivität der aufeinanderfolgenden Wertelemente umfasst, die elektrisch mit der elektrischen Schaltung gekoppelt sind.

12. Verfahren nach Anspruch 8, wobei das Berechnen des Gesamtwerts der aufeinanderfolgenden Wertelemente Berechnen der Gesamtwerte von zwei oder mehr elektrischen Eigenschaften der aufeinanderfolgenden Wertelemente umfasst, die elektrisch mit der elektrischen Schaltung gekoppelt sind.

13. Verfahren nach Anspruch 9, wobei die zwei oder mehr elektrischen Eigenschaften Widerstand und Kapazität sind.

## Revendications

1. Système pour surveiller l'utilisation d'un récipient de médicaments ayant une pluralité de cellules contenant un médicament, comprenant :
un circuit électrique comprenant
une pluralité d'éléments de valeur séquentiels couplés électriquement en parallèle entre une mise à la terre et un orifice unique, chaque élément de valeur séquentiel étant conçu pour une association avec une cellule différente de ladite pluralité des cellules contenant un médicament et ayant une valeur unique ; et
une alimentation sans fil, pour fournir de l'énergie de sorte qu'une source d'alimentation séparée est omise ; et
une pluralité de sections cassables, chaque section cassable étant conçue pour une association avec une cellule différente de ladite pluralité des cellules contenant un médicament et un élément différent de ladite pluralité d'éléments de valeur séquentiels, dans laquelle chaque section cassable est conçue pour être brisée pour accéder au médicament à l'intérieur de la cellule associée et pour découpler électriquement l'élément de valeur séquentiel associé du circuit électrique ; et
un dispositif de mesure associé à l'orifice et programmé pour calculer la valeur combinée des éléments de valeur séquentiels couplés électriquement au circuit électrique et, selon la valeur combinée, déterminer lesquelles des cellules ont été accédées par un bris de la section cassable associée.

2. Système selon la revendication 1, dans lequel au moins un élément parmi lesdits éléments de valeur séquentiels comprend un condensateur.

3. Système selon la revendication 1, dans lequel au moins un élément parmi lesdits éléments de valeur séquentiels comprend une résistance.

4. Système selon la revendication 1, dans lequel au moins un élément parmi lesdits éléments de valeur séquentiels comprend un inducteur.

5. Système selon la revendication 1, dans lequel au moins un élément parmi lesdits éléments de valeur séquentiels comprend une combinaison de composants électriques.

6. Système selon la revendication 1, dans lequel au moins un élément parmi lesdits éléments de valeur séquentiels comprend un semi-conducteur.

7. Système selon la revendication 1, comprenant en outre un second circuit électrique comprenant une pluralité d'éléments de valeur séquentiels couplés électriquement en parallèle entre une mise à la terre et ledit orifice unique, chaque élément de valeur séquentiel du second circuit électrique étant conçu pour une association avec une cellule différente de ladite pluralité des cellules contenant un médicament et ayant une valeur unique, et
une pluralité de sections cassables, chaque section cassable du second circuit électrique étant conçue pour une association avec une cellule différente de ladite pluralité des cellules contenant un médicament et un élément différent de ladite pluralité d'éléments de valeur séquentiels du second circuit électrique, dans lequel
chaque section cassable du second circuit est conçue pour être brisée pour accéder à un médicament à l'intérieur de la cellule associée et pour découpler électriquement l'élément de valeur séquentiel associé du second circuit électrique,
les circuits électriques sont couplés électriquement en parallèle, et
au moins un parmi les circuits électriques comprend un transistor positionné entre les éléments de valeur séquentiels desdits au moins un parmi les circuits électriques et une mise à la terre.

8. Procédé de surveillance de l'utilisation d'un médicament, comprenant :
l'association d'un circuit électrique et d'un récipient de médicaments ayant une pluralité de cellules contenant un médicament de sorte que chaque cellule contenant un médicament comprend un élément de valeur séquentiel associé du circuit électrique et une section cassable associée, avec chaque élément de valeur séquentiel ayant une valeur unique et étant électriquement couplé au circuit électrique par la section cassable associée qui, lorsque brisée, découple l'élément de valeur associé du circuit électrique et donne accès au médicament à l'intérieur de la cellule associée ;
le calcul de la valeur combinée des éléments de valeur séquentiels couplés électriquement au circuit électrique et, selon la valeur combinée, la détermination des cellules auxquelles on a accédé par un bris de la section cassable associée ; et
l'obtention d'une alimentation sans fil, pour fournir de l'énergie de sorte qu'une source d'alimentation séparée est omise.

9. Procédé selon la revendication 8, dans lequel ledit calcul de la valeur combinée des éléments de valeur séquentiels comprend le calcul de la capacitance combinée des éléments de valeur séquentiels couplés électriquement au circuit électrique.

10. Procédé selon la revendication 8, dans lequel ledit calcul de la valeur combinée des éléments de valeur séquentiels comprend le calcul de la résistance combinée des éléments de valeur séquentiels couplés électriquement au circuit électrique.

11. Procédé selon la revendication 8, dans lequel ledit calcul de la valeur combinée des éléments de valeur séquentiels comprend le calcul de l'inductance combinée des éléments de valeur séquentiels couplés électriquement au circuit électrique.

12. Procédé selon la revendication 8, dans lequel ledit calcul de la valeur combinée des éléments de valeur séquentiels comprend le calcul des valeurs combinées de deux propriétés électriques ou plus des éléments de valeur séquentiels couplés électriquement au circuit électrique.

13. Procédé selon la revendication 9, dans lequel lesdites deux propriétés électriques ou plus sont la résistance et la capacitance.
